# EUROPEAN PATENT APPLICATION

(11) **EP 4 817 588 A1**
(43) Date of publication of application: **07.10.2026**
(21) Application number: 26163634.4
(22) Date of filing: 10.03.2026
(51) Int. Cl.: A61J 15/00, A61J 7/00, A61J 1/10

(54) **A SYSTEM FOR COOLING BREAST MILK, A HOUSING WITH A COOLING SYSTEM, AND A METHOD FOR COOLING BREAST MILK**

(30) Priority: 31.03.2025 US 202519095191
(71) Applicant: Medela AG, 6340 Baar (CH)
(72) Inventor: Keclik, James, McHenry, IL 60051 (US)
(74) Representative: Pesce, Michele

(57) **Abstract**

System for providing a newborn with breast milk, including a container fluidly connected to a tubing system to convey breast milk to the newborn, a dispensing device to convey the breast milk from the container through the tubing system to the newborn, and a cooling system including a cooling device to cool the breast milk. A housing to support a container to receive breast milk and fluidly connected to a tubing system to convey breast milk through a dispensing device to a newborn, the housing having a cooling system including a cooling device to cool the breast milk. A method for providing breast milk to a newborn, wherein a container fluidly connected to a tubing system receives breast milk, and the breast milk is conveyed from the container through the tubing system through a dispensing device to the newborn, and the breast milk is cooled while contained in the container.

## Description

### FIELD OF THE INVENTION

The present disclosure relates to a system for providing a newborn with breast milk, a housing adapted to receive a cooling system, as well as a method for providing breast milk to a newborn.

### BACKGROUND

Premature infants, i.e. those born before a gestational age of 37 weeks, multiple-birth infants, such as twins or triplets, and other newborns facing early health challenges, typically spend the initial phase of their lives in a neonatal intensive care unit, or "NICU". Here, these newborns are able to gain strength and grow, while being provided with continuous care and monitoring by specifically trained medical staff. However, in most neonatal intensive care units, the newborn is separated from the mother, especially since most mothers are discharged from the corresponding hospital well before their newborn. This presents a practical challenge in providing the breast milk produced by the mother to the newborn. The breast milk must not only be expressed from the mother's breast, but collected, stored, transported, and conveyed to the newborn, all while maintaining the nutritional and safety integrity of said breast milk.

While separated from their newborn, the mother typically expresses and collects the produced breast milk in specific collection receptacles provided with identifying indicia, such as the identity of the mother, the identity of the newborn for whom the breast milk is intended, the volume of breast milk collected, and the time and date of collection, for example. The collected breast milk is subsequently cooled to typical refrigerator temperatures or frozen. After gathering a certain amount of collection receptacles containing breast milk, these are then brought to the hospital or medical clinic for feeding to the newborn. Dependent on the age and medical condition of the newborn, provision of breast milk from the mother to the hospital may occur multiple times a day, daily, every few days, or even weekly. Once received by the medical personnel, the breast milk is then typically stored in a cooled storage facility of the NICU, until required by the newborn.

The general feeding schedule of a newborn requires the conveyance of breast milk to the newborn approximately every three hours. Consequently, the responsible healthcare professional collects the breast milk to be fed to the newborn, confirming that the breast milk corresponds to said newborn and the other identifying indicia are correct. Thereafter, they may prepare a fortified mixture with the breast milk to increase at least one or more of the fat, protein, and carbohydrate contents thereof, prior to transferring the breast milk to a dispensing system operatively and fluidly connected to an enteral feeding pump, for providing the newborn with the breast milk. An enteral feeding pumping is generally required, as the newborn has not yet developed the ability to feed orally, due to the complex suck-swallow-breathe mechanism it has not yet developed. The enteral feeding pump is then manually programmed by the healthcare professional to personalize the required feeding program for the specific newborn being fed. Such a feeding program is typically prescribed and ordered by the attending physician. Once the breast milk has been conveyed to the newborn, the health care professional disengages the newborn from the enteral feeding pump and prepares the enteral feeding pump for use for a different newborn.

The temperatures the breast milk is exposed to between expression from the mother's breast to feeding thereof to the newborn play a critical role in ensuring that the breast milk not only exhibits the required nutritional value but does not provide new pathogens that may worsen the newborn's medical situation. Consequently, once the breast milk is expressed from the mother's breast, the breast milk is generally directly refrigerated or frozen, prior to being brought to the hospital or clinic, where it is then typically stored in a cooled storage facility of the NICU. Thereafter, the responsible healthcare professional gathers the breast milk corresponding to the newborn to be fed, may prepare a fortified mixture therewith to increase at least one or more of the fat, protein, and carbohydrate contents of said breast milk, and provides the breast milk to the newborn in a single feeding session. Generally, the breast milk is provided to the newborn via an enteral feeding system, as the newborn has not yet developed the ability to feed orally, due to the complex suck-swallow-breathe mechanism it has not yet developed.

Nonetheless, between the removal of the breast milk from the cooled storage area of the NICU until the feeding thereof to the newborn, there remains risk that the temperature of the breast milk and the duration at said temperatures exceeds health standard guidelines, reducing the acceptable volume of milk that may be prepared and fed to the newborn. By continuously checking the temperature of the breast milk during these preparation and feeding steps and requiring intense monitoring of the breast milk conditions, the feeding process becomes lengthy and tedious for the medical staff, extending the duration of each feeding session pro newborn.

Consequently, the present invention aims to provide a system for providing a newborn with breast milk, in addition to a corresponding housing and method thereto, that may reduce the risk of spoiled breast milk by enabling further cooling prior to the conveying of breast milk to the newborn, e.g. when the breast milk is contained in the container but is not conveyed to the newborn.

According to a first aspect of the present invention there is provided a system for providing a newborn with breast milk, comprising a container adapted to received said breast milk and fluidly connected to a tubing system to convey the breast milk to the newborn, a dispensing device adapted to convey the breast milk from the container, through the tubing system and to the newborn, and a cooling system including a cooling device adapted to cool the breast milk.

The tubing system conveys the breast milk from the container to the newborn and may physically define the flow path of the breast milk from the container to the newborn. The tubing system may include at least one flexible tube that may be constructed of polyvinyl chloride (PVC), polyurethane, or silicone. The tubing system may be directly coupled with the container. The tubing system may further comprise a nasogastric or orogastric tubing section to be introduced to the newborn's respective anatomy.

The container may be a rigid article, such as commonly associated with a breast milk bottle or syringe dispenser, or a flexible component, such as a breast milk bag or pouch. Preferably, the container is either a breast milk bag or a syringe dispenser. With respect to the latter, a syringe dispenser may include a plunger that tightly fits into a cylindrical barrel of said syringe dispenser; however, a syringe dispenser does not necessarily require a plunger.

The dispensing device is adapted to convey the breast from the container, through the tubing system, and to the newborn. In particular, the dispensing device may comprise a pump, to achieve such conveyance of the breast milk. The dispensing device may provide forces responsible for extracting the breast milk from the container, conveying the breast milk through the tubing system and providing said breast milk to the newborn. The dispensing device may be operatively connected to the tubing system.

The cooling system, which includes a cooling device, is adapted to cool the breast milk, preferably the breast milk received in the container. This may be achieved by removing the heat contained within the breast milk via a heat pump, for example. As such, the cooling device is preferably realized by a heat pump, such as a thermoelectric heat pump that implements the Peltier effect to transfer heat from a first side to a second side, thereby cooling and heating said sides, respectively. The cooling system may be adapted to cool the breast milk between 0 and 15 °C, preferably between 1 and 10 °C, and more preferably between 2 and 5 °C; e.g. the breast milk is cooled at a temperature of 4 °C.

By incorporating the container with breast milk into a system comprising components to convey the breast milk from said container to the newborn, i.e. the tubing system and the dispensing device, in addition to a cooling system, an additional cooling phase of the breast milk may be established prior to and possibly during feeding said breast milk to the newborn. This may prevent further warming or even counteract any warming the breast milk underwent after removal from the cooled storage facility in the NICU. This may extend the lifetime of the breast milk and consequently allow the continued use thereof for the scheduled feeding session.

The system may further comprise a control unit for controlling the operation of the cooling system. The control unit may be operatively connected to the cooling system by means of a wired or wireless connection.

Furthermore, the system may also comprise a user interface operatively connected to the control unit. The control unit may be operatively connected to the user interface by means of a wireless or wired connection.

The user interface may be adapted to receive input, preferably from a user, and provide said input to the control unit, in order to control the operation of the cooling system. More specifically, input provided over the user interface may instruct the control unit to set at least one cooling parameter of the cooling system. Preferably, input may be provided over the user interface prior to and during operation of the system, so that the input over the user interface may not only set at least one of the cooling parameters but may adjust at least one of the cooling parameters during use of the system. As such, the control unit may be adapted to set and adjust at least one of the cooling parameters of the cooling system. Therefore, cooling parameters may be modified and adjusted during the conveyance of breast milk from the container, through the tubing system, and to the newborn. Moreover, the user may also set the cooling parameters prior to the operation or use of the system, i.e. prior to breast milk being conveyed from the container.

The user interface may comprise at least one or more of a touch screen, a setting dial, and a press button. Moreover, the user interface may further comprise a display that may display a least one or more of the set cooling parameters, real-time information regarding the cooling parameters, and patient information of the newborn, amongst others.

A cooling parameter is preferably at least one or more of a cooling temperature and a cooling duration.

A cooling temperature may be defined as the temperature to which the breast milk is cooled. A cooling temperature may be between 0 and 15 °C, preferably between 1 and 10 °C, more preferably between 2 and 5 °C and e.g. the breast milk is cooled at a temperature of 4 °C.

A cooling duration may be defined as a time period during which the breast milk is cooled by the cooling system. A cooling duration may be represented as a time interval, such as between 1 and 12 hours, preferably between 2 and 6 hours, more preferably 3 or 4 hours. A cooling duration may also be continuous and uninterrupted. Such a continuous cooling duration may reflect the time period during which the container contains breast milk.

Furthermore, a control unit may also be operatively connected to the dispensing device for controlling the operation of said dispensing device. The control unit may be operatively connected to the dispensing device by means of a wireless or wired connection. Moreover, such a control unit may also be operatively connected to a user interface.

Input provided over a user interface may instruct the control unit to set at least one dispensing parameter of the dispensing device. Preferably, the control unit is also adapted to adjust at least one dispensing parameter of the dispensing device, preferably during use thereof. Consequently, input over the user interface may set and adjust at least one dispensing parameter of the dispensing device during implementation of the dispensing device, preferably during use of the system. This would allow for the use modification and adjustment of dispensing parameters prior to and during the conveyance of breast milk from the container, through the tubing system, to the newborn. Input over the user interface may also set at least one dispensing parameter prior the operation or use of the dispensing device or system.

A dispensing parameter may be at least one or more of a dispensing time and a breast milk amount.

The dispensing time may be defined as the duration during which breast milk is conveyed from the container to the newborn, i.e. a period during which the newborn receives the breast milk. A dispensing time may be between 1 and 60 minutes, preferably between 10 and 50 minutes, more preferably between 20 and 40 minutes and more preferably 30 minutes.

A breast milk amount may be defined as the amount of milk provided to the newborn, which may be associated with a single feeding session. Although preferably given as a volumetric unit of measure, the breast milk amount may also be provided as a mass value.

A user interface operatively connected to the dispensing device may comprise a display to display a least one or more of the set dispensing parameters, real-time information regarding the dispensing parameters, and patient information of the newborn, amongst others.

In a further embodiment of the invention, the system may comprise a housing adapted to support the container. In order to do so, the housing may comprise at least one support on an outer surface of the housing to support the container. Such a support may comprise at least one or more of a bracket, a hook, a peg, and similar mounting system components.

In an alternative embodiment, the housing may exhibit a recess to accommodate the container. The recess may be formed to reflect the general shape of the container, especially the outer boundary contour of the said container, preferably when filled with breast milk. Moreover, the recess may include at least one support, on which the container may be placed or hung, for example. Such a support may carry the weight of the container. Preferably, a support may be realized by a hook, an arm, or a peg, for example.

The housing may further include a cover associated with the recess so that the recess and the cover forms a boundary of a closed compartment adapted to accommodate the container. This may represent a closed position of the cover and the recess. Together, the recess and cover establish an outer boundary such that the container may be fully encompassed by the housing within said closed compartment. Preferably, the shape of the closed compartment reflects the general contour of the container, in particular the general contour of the container with breast milk, preferably filled with breast milk.

The cover, which may be a separate component from the housing, may be attached thereto via a latching system or a hinge or sliding construction. The latter may allow the user to open the closed compartment with one hand, thereby enabling ease of use.

Preferably, when in a closed position, the cover and the recess establish a sealed closed compartment. This may establish a sealed atmospheric environment in the closed compartment.

Preferably, the closed compartment is thermally insulated, especially when in a closed position.

Preferably, the cover may include window or be constructed of a transparent material, such as polycarbonate, Poly(methyl methacrylate) (PMMA), or polyethylene terephthalate (PET). Such a construction allows for a quick visual verification of the fill-status of the container, in addition to other information provided on said container, without the need to open the closed compartment or handle the container during use thereof.

Furthermore, the cover of the housing may include at least one aperture through which the tubing system may pass. Preferably, such an aperture includes a sealing component, such as a sealing ring, in order to ensure that the closed compartment remains sealed against the outside environment of the housing. Such a sealing component may also provide thermal insulation.

In a further embodiment of the system the cooling system may further comprise a compartment cooling means adapted to cool a closed compartment of the housing. The compartment cooling means may cool at least one of the boundary walls of the recess, preferably the entire recess and more preferably the entire closed compartment.

Preferably, the container is accommodated in the closed compartment during cooling thereof, in order to cool the breast milk received therein. However, the closed compartment may be cooled by the compartment cooling means prior to insertion of the container into the closed compartment. This may help ensure that the temperature of the breast milk does not rise during initialization of the cooling by the compartment cooling means.

At least a portion of the tubing system, in particular a section connected to the container and accommodated in the closed compartment, may be cooled by the compartment cooling means.

In a further embodiment of the system, the cooling system may comprise a breast milk cooling means including a cooling line fluidly connected to the container and a pumping device for circulating the breast milk from the container, through the cooling line, to the cooling device, and to the container.

Preferably, the cooling line is a re-circulation line that is fluidly connected to the container at an inlet and fluidly connected to the container at an outlet, wherein the breast milk is circulated through the cooling line from the inlet to the outlet. The cooling line may therefore physically define the flow path of the breast milk in the breast milk cooling means. The cooling line may include at least on flexible tube that may be constructed of polyvinyl chloride (PVC), polyurethane, or silicone.

The cooling device is preferably located along the cooling line. This may allow for breast milk flowing passed the cooling device to be cooled prior to being re-introduced into the container.

Preferably, the pumping device is operatively connected to the control unit by means of a wireless or wired connection, for controlling the operation of said pumping device. Moreover, such a control unit may also be operatively connected to a user interface.

In particular, a user interface may be adapted to receive input, preferably from a user, and provide said input to the control unit, in order to control the operation of the pumping device. More specifically, input provided over the user interface may instruct the control unit to set a least one pumping parameter of the pumping device. Preferably, input may be provided over the user interface prior to and during operation of the system, so that the input over the user interface may not only set at least one of the pumping parameters but may adjust at least one of the pumping parameters during use of the system. As such, the control unit may be adapted to set and adjust at least one of the pumping parameters of the pumping device. Therefore, pumping parameters may be modified and adjusted during the conveyance of breast milk from the container, through the tubing system, and to the newborn. Moreover, the user may also set the pumping parameters prior to the operation or use of the system, i.e. prior to breast milk being conveyed from the container.

Preferably, a pumping parameter may be at least one or more of a breast milk flow rate and a pumping duration.

A pumping duration may be defined at the time period during which breast milk is circulated through the breast cooling means. A pumping duration may be represented as a time interval, such as between 1 and 12 hours, preferably between 2 and 6 hours, more preferably 3 or 4 hours. A pumping duration may also be continuous and uninterrupted. Such a continuous pumping duration may reflect the time period during which the container contains breast milk.

The cooling system may further comprise at least one temperature sensor operatively connected to a control unit. Specifically, for an embodiment of the cooling system comprising a compartment cooling means, the temperature sensor may be located within the closed compartment, preferably on a wall of the recess. With respect to an embodiment of the cooling system comprising a breast cooling means, the temperature sensor may be located along the cooling line, preferably along a section of the cooling line passed the cooling device.

Preferably, a control unit operatively connected to the cooling system further comprises a processer that is adapted to receive and record the temperature values measured by the temperature sensor of the cooling system. Moreover, the processor may be adapted to compare said values with provided control values, in order to establish a feedback mechanism. Such a feedback mechanism may allow for the control and confirmation that the breast milk is cooled to temperatures that correspond to the instructions provided to the control unit for controlling the operation of the cooling system. The temperatures detected by the temperature sensor may be displayed on a display of the user interface in real-time. Preferably, the temperature sensor measures the temperature continuously, although interval measurements may also be done.

Preferably, the system comprises a single user interface operatively connected to at least one, preferably all of, a control unit for controlling the operation of the cooling system, a control unit for controlling the operation a dispensing device, and a control unit for controlling the operation of a pumping device. Preferably, the system comprises a single control unit that is adapted to control the operation of the at least one, more preferably all of, the cooling system, a dispensing device, and a pumping device.

The housing according to any embodiment of the invention may contain or house at least one or more of the cooling system and the dispensing device.

With respect to an embodiment of the invention including a breast milk cooling means, a housing may preferably support the container and house or contain at least one or more, preferably all, the cooling device, the cooling line, and the pumping device. More preferably, the housing also accommodates the container.

The cooling system may include both a compartment cooling means and a breast milk cooling means. In such an embodiment, at least one of the cooling parameters of the compartment cooling means may differ from that of the breast cooling means. For example, the cooling temperature of the compartment cooling means may be warmer than the cooling temperature of the breast milk cooling means.

According to a second aspect of the present invention, there is provided a housing adapted to support a container, wherein said container is adapted to received breast milk and is fluidly connected to a tubing system to convey said breast milk to a newborn. Moreover, the housing is also adapted to receive a cooling system that includes a cooling device adapted to cool the breast milk.

According to a third aspect of the present invention, there is provided a method of providing beast milk to a newborn, wherein breast milk is stored in a storage facility, wherein a container that is fluidly connected to a tubing system receives the breast milk, and wherein said breast milk is conveyed from the container, through the tubing system to the newborn. Prior to being conveyed to the newborn, the breast milk is transported from the storage facility to a system for providing a newborn with breast milk. Said system comprises the container, the tubing system, a dispensing device adapted to convey the breast milk from the container through the tubing system to the newborn, and a cooling system that includes a cooling device and is adapted to cool the breast milk. Furthermore, the breast milk is cooled by the system prior to being conveyed to the newborn.

The breast milk stored in a storage facility is preferably simultaneously cooled in the said storage facility. Preferably, the breast milk may be maintained at a temperature between 0 and 15 °C, preferably between 1 and 10 °C, and more preferably between 2 and 5 °C, during storage and e.g. the breast milk is cooled at a temperature of 4 °C.

The breast milk stored in the storage facility may be received in a storage receptacle prior to being received in the container fluidly connected to the tubing system. In such an embodiment, the storage receptacle differs from the container and the breast milk may be transferred from the storage receptacle to the container prior to conveyance thereof to the newborn. During such a transfer step, the breast milk may be supplemented with fortifiers or other nutritional supplements that may increase at least one or more of the fast, protein, and carbohydrate content of the breast milk.

Alternatively, the breast milk may be received in the container and subsequently stored therein in the storage facility. The addition of nutritional supplements and fortifiers may occur before or after storage of the breast milk received in the container.

The transportation of the breast milk to the system for conveyance to the newborn may refer to the removal of the breast milk from the storage facility and the provision thereof to the system. Providing the breast milk to the system may include operatively connecting the container to the cooling system, so that the breast milk received therein may be cooled. Providing the breast milk to the system may also further include fluidly connecting the container to the tubing system and/or operatively connecting the tubing system to the dispensing device.

Cooling of the breast milk by the system prior to said breast milk being conveyed to the newborn may refer to the breast milk received in the container being cooled prior to reaching the newborn. Furthermore, conveying breast milk from the container may refer to the extraction or removal of breast milk from said container and the subsequent flow of the breast milk in the direction of the newborn via the tubing system. Therefore, cooling breast milk prior to being conveyed from the container may refer to the breast milk being cooled before exiting the container. Preferably, the breast milk is cooled prior to entering the tubing system.

Preferably, the system cools the breast milk to a temperature between 0 and 15 °C, preferably between 1 and 10 °C, and more preferably between 2 and 5 °C.

Moreover, an amount of breast milk remaining in the container may be cooled while a different amount of breast milk is being conveyed from the container to the newborn. Preferably, breast milk received in the container is cooled independent of an amount of breast milk being conveyed to the newborn. Preferably, the breast milk in the container continues to be cooled while an amount of breast milk is being conveyed from the container through the tubing system to the newborn. As such, the cooling of breast milk received in the container and conveyance of breast milk from the container may occur simultaneously, preferably, independent from one another.

Cooling the breast milk after its removal from the storage facility and prior to conveying it to the newborn may ensure an improved stability and preservability of the breast milk. Moreover, the cooling of the breast by the system may counteract any warming effects undergone after removal from the storage facility, especially if cooling was implemented by the storage facility. In such an embodiment of the invention, the cooling of the breast milk received in the container may be considered a second cooling step, wherein the first cooling step is implemented during storage in the storage facility.

The cooling of the breast milk may be segmented into at least two cooling phases, wherein a first cooling phase may exhibit a different cooling temperature than that of a second cooling phase. Moreover, the at least two cooling phases may manifest different cooling durations. Specifically, the cooling system may be instructed to cool the breast milk in at least two cooling phases, wherein at least one of the cooling parameters for at least one of the cooling phases differs. For example, a control unit may instruct the cooling system to cool the breast milk at a lower temperature for a first cooling phase and a warmer temperature during a second cooling phase.

In a further embodiment, the cooling system comprise a breast milk cooling means that removes the breast milk from the container and transports the breast milk through a cooling line to a cooling device, whereby the breast milk is cooled. The cooled breast milk is then transported further along the cooling line back to the container. Preferably, a pumping device pumps the breast milk from the container, through the cooling line, and back to the container. Preferably, the cooling line is a re-circulation loop.

Preferably, the container is directly connected to the tubing system. Furthermore, the tubing system is preferably connected to a dispensing device, which ensures that the breast milk is conveyed from the container through the tubing system and to the newborn. The tubing system may be operatively connected to the dispensing device prior to fluidly connecting the tubing system with the container.

This invention may also be said broadly to consist in the parts, elements and features referred to or indicated in the specification of the application, individually or collectively, and any or all combinations of any two or more said parts, elements or features, and where specific integers are mentioned herein which have known equivalents in the art to which this invention relates, such known equivalents are deemed to be incorporated herein as if individually set forth. As used herein "(s)" following a noun could mean the plural and/or singular forms of the noun.

Further details, advantages or features of the present invention will become apparent from the following description in combination with the figures. Examples of embodiments of the invention will be further described with reference to the figures, in which,

### BRIEF DESCRIPTION OF THE SEVERAL VIEWS OF THE DRAWINGS

Figure 1 illustrates a perspective view of a first embodiment of a system;
Figure 2 illustrates an additional perspective view of a first embodiment of a system;
Figure 3 illustrates a perspective view of a second embodiment of a system:
Figure 4 illustrates a side-view of a container on a support;
Figure 5 illustrates a perspective view of an embodiment of a system with a cooling system and an agitation system;
Figure 6 illustrates a side-view of a container accommodated within a housing and connected to a re-circulation arrangement of an agitation system;
Figure 7a illustrates a side-view of a container and a vibration means of a container agitation means accommodated within a housing;
Figure 7b illustrates a side-view of a container and a vibration means of a container agitation means accommodated within a housing;
Figure 8a illustrates a side-view of a container and a reciprocating piston of a container agitation means accommodated within a housing;
Figure 8b illustrates a side-view of a container and two reciprocating pistons of a container agitation means accommodated within a housing;
Figure 9a illustrates a side-view of a container and a rotating cam of a container agitation means accommodated within a housing; and
Figure 9b illustrates a side-view of a container and two rotating cams of a container agitation means accommodated within a housing.

### DETAILED DESCRIPTION OF THE PREFERRED EMBODIMENTS

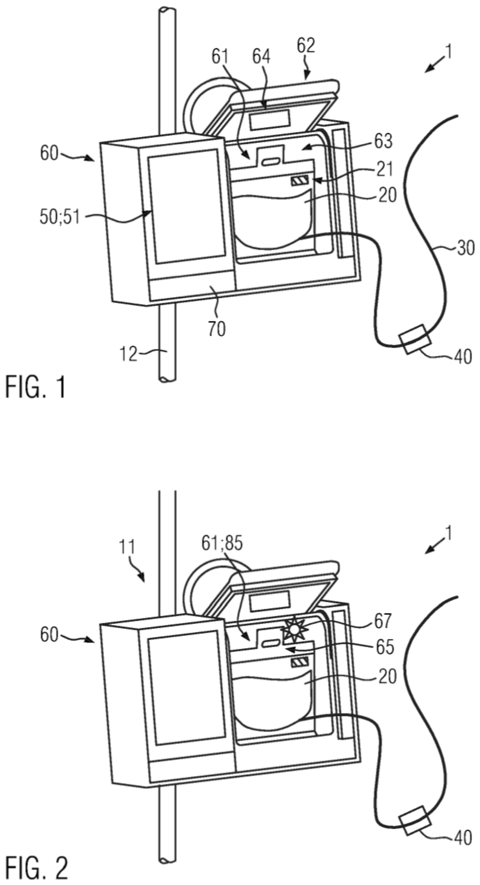

A first embodiment of a system 1, as illustrated in Figures 1 and 2, manifests a container 20 fluidly connected to a tubing system 30 and a dispensing device 40 adapted to convey breast milk from the container 20 through the tubing system 30 to a newborn; e.g. the dispensing device is a pump such as a volumetric pump. A warmer (not shown) may be provided on the tubing system 30, to warm the breast milk to a temperature adapted for feeding the breast milk to the newborn. The container 20, which is depicted as a breast milk bag, is adapted to receive multiple batches of breast milk, while the dispensing device 40 is adapted to provide at least one of said batches of breast milk from said container 20 to the newborn.

The container 20 is supported by support 65 located within a recess 61 of housing 60, wherein said recess 61 is closed by a cover 62 with a window 64. Together, the recess 61 and the cover 62 form a boundary of a closed compartment 63, which surrounds and accommodates the container 20. The window 64 of the cover 62 enables a user to optically review and identify the amount of breast milk within the container 20 without the necessity of opening the cover 62 to the closed compartment 63, in order to do so. Moreover, the cover 62 exhibits an aperture through which the tubing system 30, which is directly connected to the container 20, may pass, allowing for said cover 62 to remain closed during use of the system 1.

The housing 60 of the system 1 further comprises a user interface 51 constructed as a touch screen with a display and operatively connected to a control unit 50, which in turn is operatively connected to the dispensing device 40 for controlling the operation thereof. The user interface 51 allows the provision of input, in order to set and adjust at least one dispensing parameter, such as dispensing time of a batch of breast milk, a breast milk amount of a batch, and an interval time between two subsequent batches, of the dispensing device 40. Moreover, the display of the user interface 51 displays the currently implemented dispensing parameters, as well as further settings, including the conditions of the closed compartment 63 and user data, for example.

Moreover, the system 1 also includes a reader 70 positioned on the housing 60, wherein said reader 70 is adapted to identify an identifier 21 located on the container 20. The identifier 21, which may be realized as a QR-code, NFC tag, or RFID tag, provides the reader 70 with information regarding the breast milk associated with the container 20, in addition to predefined dispensing parameters. Since the reader 70 is operatively connected to the control unit 50, the data provided by the identifier 21 to the reader 70 can directly instruct the control unit 50 without the need to input said parameters via the user interface 51.

The housing 60 of the first embodiment not only exhibits a carrying handle 11 to enable simplified transportation and re-location thereof, but also an attachment means to secure the housing 60 to a stand 12, such as an IV pole. The attachment means is a two-handed clamp mechanism activated by the dual-depression of two buttons, which removes the clamp force from the stand 12 so that the housing 60 may be installed or removed therefrom.

As illustrated in Figure 2, the recess 61 of the housing 60 includes an illumination component 67 that provides light to the closed compartment 63. The illumination component 67 is operatively connected to the user interface 51 and control unit 50, so that the illumination of the closed compartment 63 or the recess 61 can be manually set and adjusted via user input over the user interface 51. Additionally, the illumination component 67 is also operatively connected to the cover 62 in such a way that positioning the cover 62 into an open position, such as that depicted in Figure 2, results in the illumination of the recess 61 and the container 20 accommodated therein.

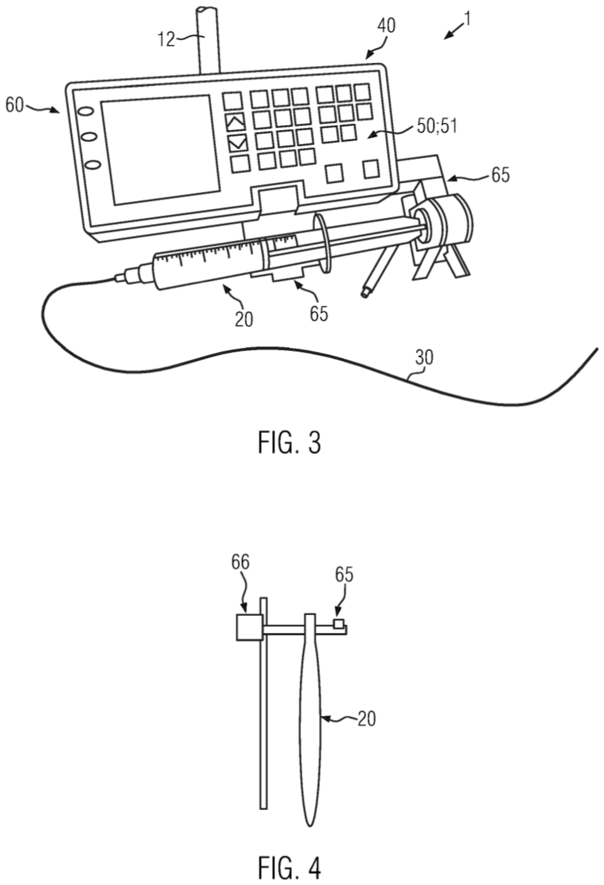

Figure 3 illustrates a second embodiment of the system 1, wherein the supports 65 of the housing 60 that support and accommodate the container 20 project from a bottom side of said housing 60. In said embodiment, the container 20 is realized by a syringe dispenser, which is fluidly connected to the tubing system 30.

As depicted in the embodiment according to Figures 1 and 2, as well as the embodiment of Figure 3, at least two preferably at least four batch amounts of breast milk are provided to and received in the container 20. Subsequently, the container 20 is fluidly connected to the tubing system 30, which is operatively connected to the dispensing device 40, and placed on the supports 65 of the housing 60. Thereafter, the tubing system 30 is fluidly connected to the newborn.

The user than provides input over the user interface 51 regarding the dispensing time of the breast milk batches, a breast milk amount of each batch, and an interval time between two subsequent batches. Subsequently, the control unit 50 instructs the dispensing device 40 accordingly and the breast milk is conveyed from the container 20, through the tubing system 30, and to the newborn in a batch manner. The container 20 remains connected to the tubing system 30 during the conveyance of all of the at least two, preferably at least four, more preferably all, batches of breast milk received in the container 20. Moreover, the container 20 is not refilled during the conveyance of said batches of breast milk or between subsequent batches. The dispensing device 40 retains the remaining batches of breast milk in the container 20 during the interval time of the batched conveyance, so that no breast milk is conveyed from the container 20 or through the tubing system 30 between batched conveyance, i.e. during the interval time between subsequent batches. A valve (not shown) may optionally be provided on the tubing system 30 to retain the remaining batches of breast milk in the container 20 during the interval time of the batched conveyance; e.g. the valve may be provided at the outlet of the container 20 or at the entrance of the dispensing device 40 or at an intermediate position between the two.

Once all batch amounts of breast milk received in the container 20 have been conveyed and provided to the newborn, that is, once the container 20 is empty, then the tubing system 30 is disengaged from the newborn.

The system 1 as illustrated in Figure 4 further comprises a weight-detecting means, wherein the support 65 which upholds the container 20 includes a weight-detecting sensor 66. Although the side-view of the system 1 in Figure 4 depicts the first embodiment according to Figures 1 and 2, it is understood that the second embodiment as illustrated in Figure 3 may also exhibit a weight-detecting means, wherein at least one of the supports 65 is associated with a weight-detecting sensor 66.

The weight-detecting sensor 66, which is operatively connected to the control unit 50 and the user interface 51, continuously measures and records the weight of the container 20. A feedback mechanism in the control unit 50 compares the measured weight value with that of a control value to ensure that the amount of breast milk actually being conveyed by the dispensing device 40 corresponds to the value provided and instructed to the control unit 50. Furthermore, due to the sensitivity of the weight-detecting sensor 66, the system 1 is adapted to detect shock or vibration situations suggesting non-ideal conveying conditions. In such a case, the feedback mechanism of the control unit 50 would instruct the dispensing device 40 to pause further conveyance of the breast milk to the newborn and display and corresponding error message on the display of the user interface 51, so that a user may respond accordingly.

Further embodiments of the system 1 may exhibit a cooling system 80 with a cooling device 81 adapted to cool the breast milk within the container 20 to temperatures between 0 and 15 °C, preferably 1 and 10 °C, and more preferably between 2 and 5 °C and e.g. the breast milk is cooled at a temperature of 4 °C. The cooling device 81 in such embodiments may be a heat pump, preferably a thermoelectric heat pump.

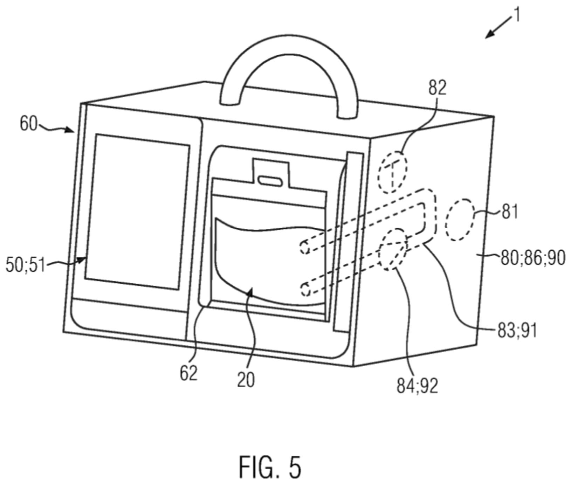

In a third embodiment of the system 1 shown in Figure 5, the cooling system 80 comprises a breast milk cooling means 86 including a cooling line 83 fluidly connected to the container 20, a pumping device 84 for circulating the breast milk from the container 20 through the cooling line 83 to the cooling device 81 and back to the container 20. The cooling device 81 in said embodiment is a thermoelectric heat pump. The individual components of said cooling system 80 are received in the housing 60 of the system 1.

The breast milk is extracted from the container 20 via the pumping device 84 and transported through the cooling line 83 to the cooling device 81, to cool the breast milk. Since the cooling line 83 is realized as a re-circulation loop, the breast milk continues to be transported from the cooling device 81 back to the container 20 via the same pumping device 84.

In a further embodiment of a system 1 including a cooling system 80, as exemplified in Figure 2, said cooling system 80 comprises a compartment cooling means 85 adapted to cool the closed compartment 63 of the housing 60. The closed compartment 63 is preferably thermally insulated. Moreover, by cooling the closed compartment 63, any container 20 with or without breast milk received in said closed compartment 63 would be cooled.

With respect to any embodiment of the invention including the cooling system 80, said cooling system 80 is operatively connected to the control unit 50 so that input over the user interface 51 can instruct the control unit 50 to set at least one cooling parameter of the cooling system 80, such as a cooling temperature and cooling duration. Moreover, in case of an embodiment with a breast milk cooling means 86, input over the user interface 51 can also instruct the control unit 50 to set at least one pumping parameter of the pumping device 84, such as breast milk flow rate and pumping duration. Input provided over the user interface 51 an also be implemented to adjust said parameters during cooling of the breast milk.

The temperature sensor 82 of an embodiment with a breast milk cooling means 86 may be located along the cooling line 83, preferably after the cooling device 81, or in a location associated with the container 20. In case of an embodiment with a compartment cooling means 85, the temperature sensor 82 may be located within the closed compartment 63 of the housing 60, preferably in the recess 61.

As depicted in Figure 5, the breast milk is pumped from the container 20 via the pumping device 84 and transported along the cooling line 83 to the cooling device 81. At the cooling device 81, the breast milk is cooled to a cooling temperature, preferably as instructed by the control unit 51, and then further transported along the remaining cooling line 83 towards the container 20. The transportation of the breast milk through the breast milk cooling means 86 may occur continuously. Specifically, a portion of the breast milk may be transported through the breast milk cooling means 86 while an amount or batch of breast milk is conveyed from the container 20 through the tubing system 30 to the newborn. The breast milk may also be transported through the breast milk cooling means 86 between the conveyance of at least two batches of breast milk.

With respect to an embodiment of the cooling system 80 comprising a compartment cooling means 85, as illustrated in Figures 1 and 2, the closed compartment 63 is cooled when the breast milk is being conveyed from the container 20 and through the tubing system 30 and between the conveyance of at least two batches of breast milk from the container 20, i.e. when no breast milk is conveyed from the container 20.

Additional embodiments of the system 1 may also encompass an agitation system 90 to agitate the breast milk.

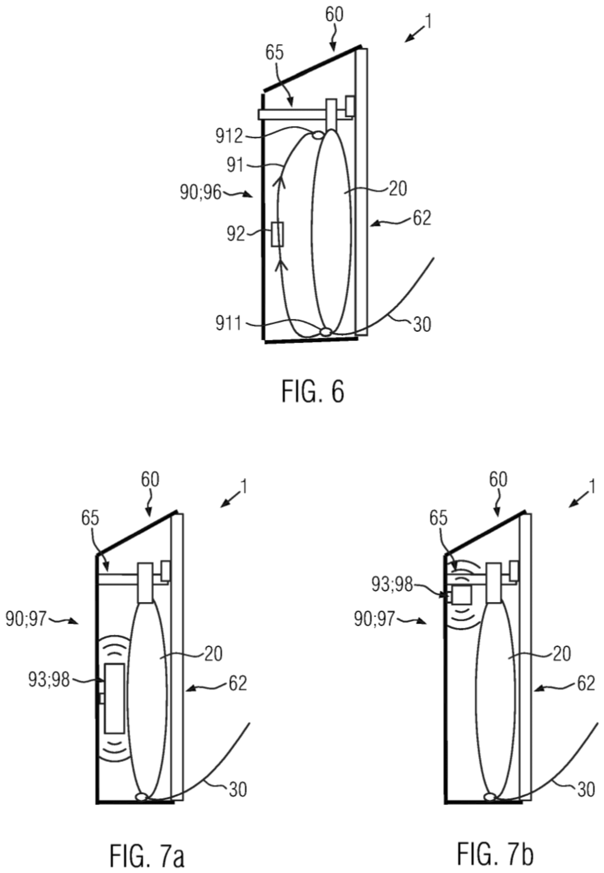

As shown in Figure 6, such an agitation system 90 may comprise a re-circulation arrangement 96, which includes a re-circulation line 91 fluidly connected to the container 20, a pumping device 92 for circulation the breast milk from the container 20 through the re-circulation line 91 and back to the container 20. The re-circulation arrangement 96 may be received in the housing 60 of the system 1.

The re-circulation arrangement 96 provides for the circulation and consequent agitation of the breast milk in the container 20 by extracting the breast milk from the container 20 via the pumping device 92, transporting the breast milk through the re-circulation line 91, and then returning the breast milk to the container 20. Preferably, the breast milk is removed from the container 20 at an inlet 911 of the re-circulation line 91 located at the bottom of the container 20 and returned at an outlet 912 of the re-circulation line 91 associated with the top of the container 20. The re-circulation arrangement 96 also results in the flow of the breast milk within the container 20.

Moreover, the agitation system 90 may comprise a container agitating means 97 that includes an actuator 98 adapted to cooperate with the container 20, in order to agitate the breast milk within said container 20.

Such an actuator 98 may comprise a vibration means 93 adapted to provide vibrational movement to the container 20. As depicted in Figure 7a, the vibration means 93 may be positioned in direct contact to the container 20, in order to ensure direct transmission of the vibrational forces to the breast milk within the container 20. However, the vibration means 93 may be in contact with the support 65 of the housing 60 supporting the container 20, as shown in Figure 7b.

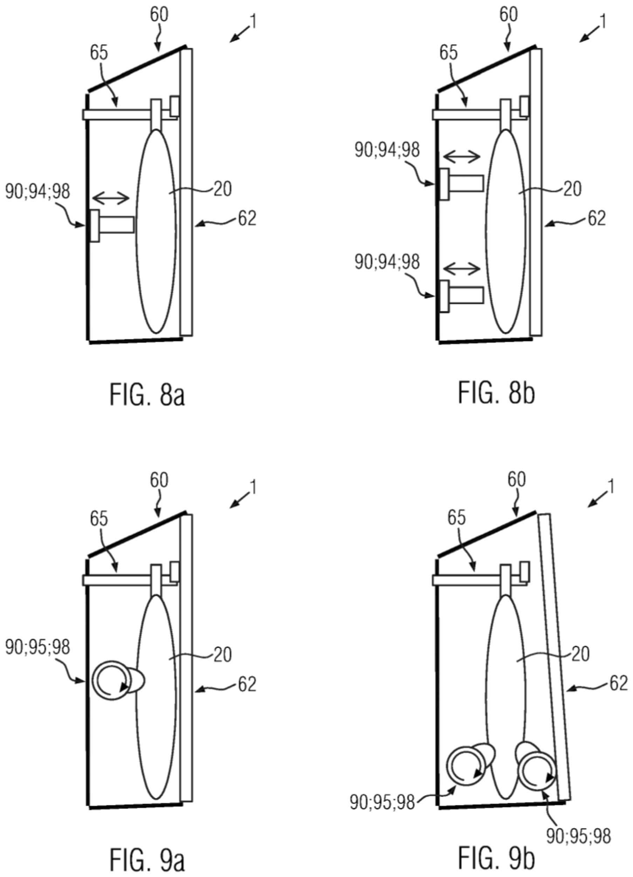

As shown in Figures 8a, 8b, 9a and 9b, the actuator 98 may also comprise at least one reciprocating piston 94 or at least one rotating cam 95. Preferably, the reciprocating piston 94 and the rotating cam 95 elastically deform the container 20 and locally display the breast milk within said container 20, thereby agitating the breast milk within the container 20. However, the reciprocating piston 94 and the rotating cam 95 may simply push on the container 20 without inducing elastic deformation, still ensuring movement of the container 20 and the breast milk therein.

The breast milk is agitated by the agitation system 90 prior to being conveyed from the container 20, through the tubing system 30, and to the newborn. Moreover, the agitation of the breast milk received in the container 20 may occur concurrently to breast milk being conveyed from the container 20, through the tubing system 30, and to the newborn. In particular, a portion of the breast milk may be transported through the re-circulation line 91, while an amount or batch of breast milk is conveyed from the container 20. Breast milk may also be transported through the re-circulation line 91 while no breast milk is being conveyed from the container 20; for example, between batched conveyance thereof. With respect to an embodiment with a container agitating means 97, the container 20 may be agitated, preferably elastically deformed, while an amount or batch of breast milk is conveyed from the container 20. The agitation system 90 may continuously agitate the breast milk received in the container 20.

In a further embodiment, the system 1 may comprise both an agitation system 90 and a cooling system 80, such that the agitation system 90 may encompass a re-circulation arrangement 96 or a container agitating means 97 housed within the closed compartment 63 of the housing, wherein the cooling system 80 comprises a compartment cooling means 85. Such a system 1 may be visualized in Figures 6 to 9b.

Alternatively, a system 1 comprising both an agitation system 90 and a cooling system 80 may exhibit a re-circulation arrangement 96 that may make use of the identical components of the breast cooling means 86. Such an embodiment is illustrated in Figure 5. This prevents the necessity of additional components and allows for the simultaneous cooling and agitation of the breast milk.

With respect to any embodiment of the invention including an agitation system 90, said agitation system 90 is operatively connected to the control unit 50 so that input over the user interface 51 can instruct the control unit 50 to set at least one agitation parameter of the agitation system 90, such as a agitation frequency and agitation duration. Moreover, in case of an embodiment with a re-circulation arrangement 96, input over the user interface 51 can also instruct the control unit 50 to set at least one pumping parameter of the pumping device 92, such as breast milk flow rate, pumping pressure, and pumping duration. Input provided over the user interface 51 an also be implemented to adjust said parameters during agitation of the breast milk.

For the purpose of the present description and of the appended claims, except where otherwise indicated, all numbers expressing amounts, quantities, percentages, and so forth, are to be understood as being modified in all instances by the term "about". Also, all ranges include the maximum and minimum points disclosed and include any intermediate ranges therein, which may or may not be specifically enumerated herein. In this context, therefore, a number A is understood as A ± 10 % of A. Within this context, a number A may be considered to include numerical values that are within general standard error for the measurement of the property that the number A modifies. The number A, in some instances as used in the appended claims, may deviate by the percentages enumerated above provided that the amount by which A deviates does not materially affect the basic and novel characteristic(s) of the claimed invention. Also, all ranges include the maximum and minimum points disclosed and include any intermediate ranges therein, which may or may not be specifically enumerated herein.

### List of Reference Signs

- 1: system
- 11: carrying handle
- 12: stand
- 20: container
- 21: identifier
- 30: tubing system
- 40: dispensing device
- 50: control unit
- 51: user interface
- 60: housing
- 61: recess
- 62: cover
- 63: closed compartment
- 64: window
- 65: support
- 66: weight-detecting sensor
- 67: illumination component
- 70: reader
- 80: cooling system
- 81: cooling device
- 82: temperature sensor
- 83: cooling line
- 84: pumping device
- 85: compartment cooling means
- 86: breast milk cooling means
- 90: agitation system
- 91: recirculation line
- 911: inlet
- 912: outlet
- 92: pumping device
- 93: vibration means
- 94: piston
- 95: cam
- 96: recirculation arrangement
- 97: container agitating means
- 98: actuator

## Claims

1. A system for providing a newborn with breast milk, comprising
a container adapted to receive said breast milk and fluidly connected to a tubing system to convey the breast milk to the newborn, and
a dispensing device adapted to convey the breast milk from the container through the tubing system to the newborn,
the system further comprises a cooling system including a cooling device adapted to cool the breast milk.

2. The system according to claim 1, the cooling system is adapted to cool the breast milk between 0 and 15 °C.

3. The system according to claim 1, wherein the cooling device is heat pump.

4. The system according to claim 1, the system further comprises a control unit for controlling the operation of the cooling system and a user interface operatively connected to the control unit,
wherein input over the user interface instructs the control unit to set at least one cooling parameter of the cooling system,
wherein a cooling parameter is at least one or more of a cooling temperature and a cooling duration.

5. The system according to claim 1, the system further comprising a housing adapted to support the container,
wherein the housing exhibits a recess to accommodate the container and further comprises a cover, wherein the cover and recess form a boundary of a closed compartment that accommodates the container, and
wherein the cooling system further comprises a compartment cooling means adapted to cool the closed compartment.

6. The system according to claim 5, the closed compartment being thermally insulated.

7. The system according to claim 5, the compartment cooling means provided with a temperature sensor operatively connected to a control unit and located within the closed compartment.

8. The system according to claim 1, the cooling system further comprises a breast milk cooling means including a cooling line which is fluidly connected to the container, a pumping device for circulating the breast milk from the container through the cooling line to the cooling device and to the container.

9. The system according to claim 8, the system further comprising a housing adapted to support the container and house the cooling device, the cooling line, and the pumping device.

10. The system according to claim 8, the breast milk cooling means is provided with a temperature sensor operatively connected to a control unit and located along the cooling line.

11. The system according to claim 8, the system further comprising a control unit for controlling the operation of the cooling system and a user interface operatively connected to the control unit,
wherein input over the user interface instructs the control unit to set at least one pumping parameter of the pumping device,
wherein a pumping parameter is at least one or more of a breast milk flow rate and pumping duration.

12. The system according to claim 1, wherein the cooling system includes both a compartment cooling means and a breast milk cooling means.

13. A housing adapted to support a container, wherein the container is adapted to receive breast milk and is fluidly connected to a tubing system to convey said breast milk through a dispensing device to a newborn,
the housing having a cooling system including a cooling device adapted to cool the breast milk.

14. A method for providing breast milk to a newborn,
wherein a container that is fluidly connected to a tubing system receives the breast milk, and
wherein said breast milk is conveyed from the container through the tubing system through a dispensing device to the newborn,
the breast milk is cooled while contained in the container.

15. The method according to claim 14, wherein breast milk received in the container is cooled, while breast milk is not conveyed from the container through the tubing system to the newborn.

16. The method according to claim 14, wherein the container is adapted to receive multiple batches of breast milk.
